# EUROPÄISCHE PATENTSCHRIFT

(11) **EP 1 210 073 B1**
(45) Veröffentlichungstag und Bekanntmachung des Hinweises auf die Patenterteilung: **26.11.2003**
(21) Anmeldenummer: 00960547.8
(22) Anmeldetag: 30.08.2000
(51) Int. Cl.: A61K 31/07, A61P 39/06, A61P 17/16

(54) **SONNENSCHUTZMITTEL ZUR ORALEN AUFNAHME**
SUNSCREEN AGENT FOR ORAL ADMINISTRATION
SUBSTANCE DE PROTECTION SOLAIRE A ADMINISTRATION ORALE

(30) Priorität: 08.09.1999 DE 19942774
(43) Veröffentlichungstag der Anmeldung: 05.06.2002
(73) Patentinhaber: Cognis Deutschland GmbH & Co. KG, 40589 Düsseldorf (DE)
(72) Erfinder: GÄRTNER, Christine, 40225 Düsseldorf (DE); STAHL, Wilhelm, 40545 Düsseldorf (DE); HEINRICH, Ulrike, 58300 Wetter (DE)
(86) Internationale Anmeldenummer: EP0008435
(87) Internationale Veröffentlichungsnummer: WO01017519

(56) Entgegenhaltungen:
- EP-A- 0 981 969
- WO-A-99/18814
- FR-A- 2 749 757
- US-A- 5 290 605
- ANONYMOUS: "LUTEIN LYCOPENE CAROTENE COMPLEX VEGICAPS" INTERNET ARTICLE, [Online] 3. März 1998 (1998-03-03), XP002125651 Gefunden im Internet: <URL:http://www.solgar.com/online_referenc e/beta_carotene/lutein.html> [gefunden am 1999-12-14]

## Beschreibung

### Gebiet der Erfindung

Die vorliegende Erfindung befindet sich auf dem Gebiet der oralen Sonnenschutzmittel und betrifft die Verwendung einer Mischung aus β-Carotin, Lutein und Lycopin.

### Stand der Technik

Unter dem Einfluss von Sonnenstrahlung kommt es zur Pigmentierung normaler Haut durch die Bildung von Melanin. Dabei ruft die Bestrahlung mit langwelligem UV-A Licht die Dunkelung der in der Epidermis bereits vorhandenen Melaninkörper hervor, ohne dass schädigende Folgen zu erkennen sind, während die kurzwellige UV-B Strahlung die Bildung neuen Melanins bewirkt. Ehe das schützende Pigment jedoch gebildet werden kann, unterliegt die Haut der Einwirkung der ungefilterten Strahlung, die je nach Expositionsdauer zu Hautrötungen (Erythemen), Hautentzündungen (Sonnenbrand) oder gar Brandblasen führen kann. Die mit derartigen Hautläsionen verbundenen Belastungen des Organismus, beispielsweise im Zusammenhang mit der Ausschüttung von Histaminen, kann zusätzlich zu Kopfschmerzen, Mattigkeit, Fieber, Herz- und Kreislaufstörungen und dergleichen führen. Daneben können Langzeitexpositionen zu kumulativer DNA-Schädigung führen, die in Hautkrebs resultieren kann. Für den Verbraucher, der sich vor den schädlichen Aspekten der Sonneneinstrahlung schützen will, stehen prinzipiell zwei Möglichkeiten zur Verfügung: zum einen der Schutz der Haut durch topische Applikation von kosmetischen Mitteln, die UV-Lichtschutzfilter enthalten, zum anderen die Erhöhung des Eigenlichtschutzfaktors der Haut durch orale Aufnahme von geeigneten Verbindungen.

Die europäische Patentanmeldung **EP 0712630 A2** (JBC Cosmetics) beschreibt eine Zubereitung zur oralen Aufnahme, die ein Carotinoid, ein Tocopherol, Ascorbinsäure sowie Selen enthält. Diese Zubereitung dient der Bräunung der Haut sowie der Prävention von Sonnenallergien (Photodermatosen). Als Carotinoide werden α-Carotin, β-Carotin sowie Lycopin in Dosen von 60 bis 150 mg pro Tag eingesetzt. Die französische Patentanmeldung **FR 2698268 A1** (L'Oréal) beschreibt eine Zusammensetzung zur oralen Aufnahme durch den Menschen, die Tryosin und/oder Phenylalanin, ein Kupfersalz sowie eine Mischung von Vitaminen enthält. Als Vitamine können Carotine, Vitamine E, Niacin sowie Vitamin C eingesetzt werden. Als Carotine werden genannt α-, β- sowie γ-Carotin und Lycopin, welches in Dosen von 5 bis 50 mg eingesetzt werden kann. Die Zubereitung dient zum Schutz der Haut vor den schädlichen Einflüssen der UV-Strahlung. Sonnenschutzmittel zur topischen Applikation, bei denen synthetischen Lichtschutzfilter durch Substanzen natürlichen Ursprungs ersetzt werden, sind in **EP 0747039 A2** (SA.FO.SA.) beschrieben. Diese Sonnenschutz-Zubereitungen enthalten eine Mischung von Aminosäuren, Vitaminen und/oder Provitaminen, Nucleoderivaten sowie Gemüseextrakten und sind in Form von Gels, Cremes oder Ölen einsetzbar. Die internationale Anmeldung **WO 97/47278** (Laboratoires Oenobiol.) beansprucht eine Mischung zur oralen Aufnahme enthaltend (a) zumindest ein natürliches Carotinoid mit Provitamin A Charakter (entweder α- oder β-Carotin), (b) zumindest ein natürliches Carotinoid ohne Provitamin A Charakter (Lycopin) sowie (c) ein weiteres Carotinoid, ausgewählt aus der Gruppe Zeaxanthin, Cryptoxanthin und Lutein, wobei das Verhältnis von (a) zum (b) 0,95:1 bis 1:50 beträgt. Diese Anmeldung beschreibt in Beispiel 1 eine Zusammensetzung aus 2,86 mg β-Carotin und 3 mg Lycopin. Die Mischung enthält weiterhin 0,07 mg Lutein als Nebenkomponente der β-Carotinquelle.

Des weiteren sei auf die internationale Patentanmeldung **WO 99/18814** (Quest) hingewiesen, aus der Carotinoidmischungen und deren Verwendung als Antioxidantien bekannt sind. Gegenstand der Französischen Patentanmeldung **FR-A 2749757** (Oenobiol) ist ein Sonnenschutzmittel, das ebenfalls eine Mischung verschiedener Carotinoide enthält. In der US-Patentschrift **US 5,290,605** (Shapiro) wird schließlich ein Softdrink vorgeschlagen, welcher neben Carotinoiden zusätzlich Vitamine enthält.

Aus dem Stand der Technik sind demnach eine Vielzahl von Mitteln zur oralen Aufnahme bekannt, die den Lichtschutzfaktor der Haut erhöhen sollen. Die Mehrzahl dieser Mittel basiert auf α- bzw. β-Carotin. Seit Studien vorliegen, die eine Supplementierung von β-Carotin mit einer erhöhten Inzidenz von Lungenkrebs in Verbindung bringen (**ATBC Studie**, The New England Journal of Medicine, 1994, 330, 1029-1035 und **CARET Studie,** G.S. Omenn et al., The New England Journal of Medicine, 1996, 334, 1150-1155) besteht das Bedürfnis nach einem Ersatz bzw. einem Teilersatz von β-Carotin in bekannten oralen Lichtschutzmitteln.

Die Aufgabe der vorliegenden Erfindung hat somit darin bestanden, verbesserte Lichtschutzmittel zur oralen Aufnahme zu entwickeln. Insbesondere sollte gegenüber bekannten Lichtschutzmitteln ein Anteil von α- bzw.- β-Carotin durch andere, mindestens ebenso wirksame Stoffe ersetzt werden. Die Anforderungen an diese Ersatzstoffe sind hoch, neben der vergleichbaren oder besseren Lichtschutzwirkung muss die toxikologische Unbedenklichkeit ebenso wie die einfache Handhabung und Formulierbarkeit der Substanzen gegeben sein. Darüber hinaus wäre es wünschenswert, wenn diese Stoffe natürlichen Ursprungs sind. Neben der Erhöhung des Lichtschutzfaktors der Haut ist es weiterhin wünschenswert, dass die Lichtschutzmittel zusätzlich die Hautalterung verzögern.

### Beschreibung der Erfindung

Gegenstand der Erfindung ist die Verwendung einer Zusammensetzung bestehend aus
(a) β-Carotin,
(b) Lutein und
(c) Lycopin
in einem Gewichtsverhältnis von (a) : (b) : (c) von 1: (0,5 -1,5) : (0,5 - 1,5) und einem für die orale Aufnahme geeigneten Träger zur Herstellung einer therapeutischen Zubereitung zur Erhöhung des Lichtschutzfaktors der Haut.

Ein weiterer Gegenstand der Erfindung betrifft die kosmetische Verwendung einer Zusammensetzung bestehend aus
(a) β-Carotin,
(b) Lutein und
(c) Lycopin
in einem Gewichtsverhältnis von (a) : (b) : (c) von 1: (0,5 -1,5) : (0,5 - 1,5) und einem für die orale Aufnahme geeigneten Träger zur Verzögerung des Alterungsprozesses der Haut.

Überraschenderweise wurde gefunden, dass durch die erfindungsgemäße orale Aufnahme der Zubereitungen eine Erhöhung des Lichtschutzfaktors der Haut erzielt wird und gleichzeitig die Hautalterung verzögert wird. Die Mischungen sind für die orale Aufnahme toxikologisch unbedenklich und hinsichtlich der Formulierung problemlos. Überraschenderweise wurde gefunden, dass gerade die Mischung dieser 3 Carotinoide in dem beanspruchten Verhältnis zueinander besonders geeignet ist, den Lichtschutzfaktor der Haut zu erhöhen sowie den Alterungsprozess der Haut zu verzögern. Im Gegensatz zu den Mischungen der WO 97/47278 zeigen die erfindungsgemäßen Mischungen eine deutlich verbesserte Erhöhung des Lichtschutzfaktors der Haut.

### β-Carotin

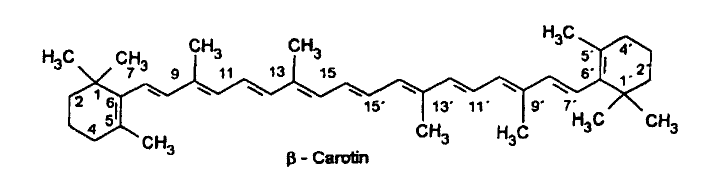

Unter β-Carotin ist ein 11-fach ungesättigtes Tetraterpen zu verstehen. Das chemische Grundgerüst besteht aus 9 konjugierten Doppelbindungen und zwei β-Ionon-Ringstrukturen an den Molekülenden, bei denen die Doppelbindungen des β-Iononsystems in Konjugation mit dem ungesättigten System der Polyenkette stehen. Die Doppelbindungen können sich in trans-Stellung befinden (trans-β-Carotin, β,β-Carotin, Provitamin A) bzw. in cis-Stellung (beispielsweise 9-cis-β-Carotin und 13-cis-β-Carotin). Unter β-Carotin im Sinne der vorliegenden Erfindung werden sowohl die cis- als auch die trans-Isomere des β-Carotins zusammengefasst. Das β-Carotin kann sowohl durch Extraktion aus pflanzlichen Quellen (beispielsweise Möhren u. anderem Gemüse, Palmöl) oder aus tierischen Materialien, Bakterien und/oder Algen (insbesondere aus der Alge *Dunaliella Salina*), auf mikrobiologischem Wege oder synthetisch über Vitamin A (Retinol) gewonnen werden. Besonders bevorzugt ist der Einsatz von β-Carotin, das durch Extraktion aus Algen gewonnen wurden, insbesondere durch Extraktion aus der Alge *Dunaliella salina*, das unter dem Handelsnamen Betatene® erhältlich ist.

### Lutein

Unter Lutein werden im Sinne der vorliegenden Erfindung sowohl Lutein [= (3R,3'R,6'R)-β,ε-Carotin-3,3'-diol; C₄₀H₅₆O_{2;} MG. 568,85] als auch die Fettsäureester des Luteins zusammengefasst. Als Fettsäureester sind Ester der Palmitinsäure, Myristinsäure, Stearinsäure, Laurinsäure sowie der Ölsäure geeignet, wobei sowohl Mono- als auch Diester in Frage kommen sowie Mischformen (wie z.B. Luteinmyristatpalmitat).

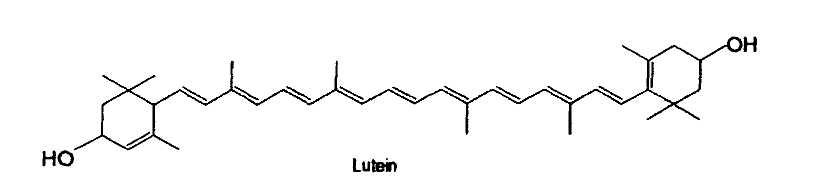

Lutein sowie seine Fettsäureester können sowohl durch Extraktion aus pflanzlichem Material (beispielsweise aus *Tagetes erecta* Arten (Studentenblume), Brennnesselblättern, Luzerne (z.B. Alfalfa), Palmöl), durch Extraktion aus tierischem Material (z.B. Eidotter) sowie aus Bakterien oder Algen gewonnen werden. Besonders bevorzugt ist Lutein, das durch Extraktion aus Pflanzen gewonnen wurden, insbesondere Lutein, das durch Extraktion aus *Tagetes erecta* Arten gewonnen wurden und unter dem Handelsnamen Xangold® erhältlich ist.

### Lycopin

Unter Lycopin werden im Sinne der vorliegenden Erfindung sowohl das all trans Isomer (ψ,ψ-Carotin, C₄₀H₅₆, MG. 536,85) als auch die cis-Isomere (wie z.B. 5-cis-, 9-cis-, 13-cis- und 15-cis Lycopin) zusammengefasst. Lycopin kann durch Extraktion aus Pflanzen (Tomate (*Solanum lycopersicum*), Hagebutte u.a. Früchten, Pfifferlingen (*Cantharellus cibarius*)) sowie durch Extraktion aus tierischem Material erhalten werden. Des weiteren kann Lycopin durch Synthese oder Extraktion aus Mikroorganismen (fermentative Gewinnung) erhalten werden. Besonders bevorzugt ist der Einsatz von Lycopin, welches durch Fermentation oder mittels Extraktion aus Pflanzen gewonnen wurde.

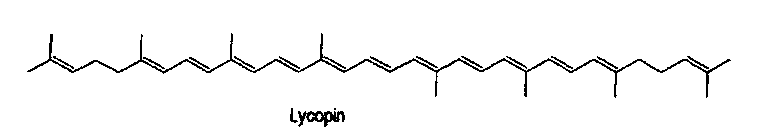

Erfindungswesentlich ist das Verhältnis der einzelnen Komponenten zueinander: Überraschender weise wurde gefunden, dass beim Vorliegen der Komponenten (a), (b) und (c) in einem Verhältnis von (a) : (b) : (c) von 1 : (0,5-1,5) : (0,5-1,5) Zubereitungen erhalten werden, die gegenüber denen des Standes der Technik eine besonders wirksame Erhöhung des Lichtschutzfaktors der Haut bewirken. Besonders bevorzugt sind Zubereitungen mit einem Verhältnis von (a) : (b) : (c) von 1 : (0,5-1,0) : (0,5-1,0), insbesondere 1,0:1,0:1,0 sowie einem Verhältnis von 1: 0,5:0,5 sowie 1: 0,75:0,75.

### Für die orale Aufnahme geeignete Träger

Ein wesentlicher Bestandteil der erfindungsgemäßen Zubereitungen ist der für die orale Aufnahme geeignete Träger. Dieser dient zum einen zum Lösen bzw. Dispergieren der erfindungsgemäßen Carotinoidmischung, vorzugsweise unterstützt er darüber hinaus die Resorption der Carotinoide aus dem Gastrointestinaltrakt. Prinzipiell sind als Träger alle Stoffe geeignet, die diese Funktionen erfüllen und toxikologisch unbedenklich sind. Beispielsweise seien hier genannt alle Speiseöle (insbesondere Sojaöl), wie Pflanzen- und Fischöle, die gegebenenfalls teilweise gehärtet sein können, des weiteren Träger auf Basis tierischer Produkte wie beispielsweise Gelatine. Des weiteren sind als Trägermaterialen beispielsweise Gummi arabicum, Saccharose, Lipide, Mono- und Diglyceride sowie Maltodextrine geeignet. Wird als Trägermaterial Wasser eingesetzt, ist die Verwendung eines geeigneten Emulgators (beispielsweise Lecitine, Sorbitanmonolaurate) üblich.

Die orale Zufuhr kann beispielsweise als Lösung, Öl, Emulsion, Suspension oder Dispersion erfolgen, geeignete Trägerformen sind beispielsweise Kapseln oder Tabletten. Üblicherweise liegen die erfindungsgemäßen Zubereitungen in Form von Weichgelatinekapseln vor. Üblicherweise werden die erfindungsgemäßen Zubereitungen hergestellt, indem man eine Vormischung aus β-Carotin, Lutein und Lycopin herstellt und diese dann zusammen mit dem Trägermaterial verkapselt. Die vorliegende Erfindung umfasst die Erkenntnis, dass die erfindungsgemäßen Zubereitungen Lebensmitteln zugesetzt werden können und die so angereicherten Lebensmittel als Träger für die orale Aufnahme dienen. Die vorliegende Erfindung umfasst weiterhin die Erkenntnis, dass den erfindungsgemäßen Zubereitungen übliche Antioxidantien, wie beispielsweise Ascorbylpalmitat (E 304), Mischtocopherole (E306), Zitronensäure (E 330) oder L-Ascorbinsäure (E 300) zugesetzt werden können.

Die Zubereitungen können zusammen mit mindestens einen weiteren Stoff verwendet werden, der ausgewählt ist aus der Gruppe, die gebildet wird von α-Carotin, Astaxanthin, α-Cryptoxanthin, β-Cryptoxanthin, Zeaxanthin, Phytoen, Phytofluen, γ-Carotin und Neurosporin. Die systematischen Namen der erwähnten Stoffe lauten wie folgt:

| | |
|---|---|
| α-Carotin | β,ε-Carotin |
| Astaxanthin | (3S,3'S)-3,3'-Dihydroxy-β,β'-carotin-4,4'-dion |
| α-Cryptoxanthin | (3R)-β,ε-Carotin-3-ol |
| β-Cryptoxanthin | (3R)-β,β-Carotin-3-ol |
| Zeaxanthin | (3R,3'R)-β,β-Carotin-3,3'-diol) |
| Phytoen | 7,8,11,12,7', 8',11',12'-Octahydro-ψ,ψ- carotin |
| Phytofluen | 7,8,11,12,7', 8',- Hexahydro-ψ,ψ- carotin |
| γ-Carotin | β,ψ-Carotin |
| Neurosporin | 7,8,-ψ,ψ-Carotin |

Besonders bevorzugt ist der Einsatz von α-Carotin.
Überraschenderweise wurde gefunden, dass durch orale Aufnahme von Zubereitungen, enthaltend (a) β-Carotine, (b) Lutein und (c) Lycopin in einem Gewichtsverhältnis von (a) : (b) : (c) von 1: (0,5 -1,5) : (0,5 - 1,5) in einem für die orale Aufnahme geeigneten Träger der Lichtschutzfaktor der menschlichen Haut erhöht wird. Zur Ermittlung des Lichtschutzfaktors der Haut sind alle dem Fachmann geläufigen Methoden geeignet, wie beispielsweise die Bestimmung der minimale Erythemwirksamkeit (MED), wie sie von der COLIPA beschrieben wird. Weitere Methoden sind die Bestimmung des Melaningehaltes sowie der Konzentration der Carotinoide in der Haut mittels Reflektionsspektrometrie und/oder HPLC sowie die chromometrische Bestimmung der Farbe der Haut (a-, b-, L-Werte). Eine Beschreibung dieser Methoden findet sich beispielsweise in **Biochemistry and Molecular Biology International 42, No. 5, 1997, S. 1023-1033.** Die Dauer der Supplementierung richtet sich üblicherweise nach dem bereits vorhandenen Lichtschutzfaktor der Haut sowie der individuell sehr unterschiedlichen Resorptionskapazität. Sie kann mehrere Tage, mehrere Wochen aber auch mehrere Monate oder Jahre durchgeführt werden. Da die erfindungsgemäßen Zubereitungen toxikologisch unbedenklich sind, ist auch eine Supplementierung von unbegrenzter Dauer möglich, wie sie beispielsweise bei Personen, die vermehrt der UV-Strahlung ausgesetzt sind, wünschenswert ist.

Überraschenderweise wurde des weiteren gefunden, dass durch orale Aufnahme von Zubereitungen, enthaltend (a), (b) und (c) in einem Gewichtsverhältnis von 1 : (0,5-1,5) : (0,5-1,5) in einem für die orale Aufnahme geeigneten Träger der Alterungsprozess der menschlichen Haut verzögert wird. Die Dauer der Supplementierung richtet sich üblicherweise nach dem Alterungszustand der Haut sowie der individuell sehr unterschiedlichen Resorptionskapazität. Sie kann mehrere Tage, mehrere Wochen aber auch mehrere Monate oder Jahre durchgeführt werden. Da die erfindungsgemäßen Zubereitungen toxikologisch unbedenklich sind, ist auch eine Supplementierung von unbegrenzter Dauer möglich.

Die Menge der Komponenten (a), (b) und (c) - ausgedrückt als Tagesdosis - liegt üblicherweise zwischen 1 und 40 mg pro Komponente, mit der Maßgabe, dass das Verhältnis von (a) : (b) : (c) bei 1 : (0,5 -1,5) : (0,5 - 1,5) liegt. Bevorzugt sind Mengen von 2 bis 25 mg pro Komponente, insbesondere von 5 bis 10 mg pro Komponente. Die erfindungsgemäßen Zubereitungen können als Einzeldosis oder in Form von Teildosen über den Tag verteilt verabreicht werden.

### Beispiele

Die Untersuchungen zur Aufnahme von Carotinoiden und zur Photoprotektion wurden mit einem Panel, bestehend aus 36 hautgesunden Probanden mit Haut des Lichttyps II, nach Fitzpatrick und Pathak durchgeführt. Zu Beginn der 12-wöchigen Untersuchung wurden die Ausgangswerte für jeden Probanden ermittelt. Eine Zwischenuntersuchung fand nach 6 Wochen statt, die Abschlussuntersuchung nach 12 Wochen. Insgesamt wurden 3 Gruppen gebildet mit jeweils 12 Teilnehmern, die folgende Tagesdosen erhiehen:

| | |
|---|---|
| 1. Gruppe | 25 mg Betatene® (entspricht 24 mg β-Carotin) |
| 2. Gruppe | 8,3 mg Betatene® (entspricht 8 mg β-Carotin), 8 mg Lycopin, 8 mg Lutein (Xangold™) |
| 3. Gruppe | Placebo-Kapseln |

Die Carotinoide wurden in Weichgelatinekapsel mit 140 mg Sojaöl verabreicht. Die Konzentration an β-Carotin, Lycopin sowie Lutein in der Haut wurde mit Hilfe der Reflektionsspektrometrie bestimmt. Diese wurden jeweils an einer Fläche von 1cm² an der Stirn, am Handrücken, an der Handinnenseite, der Unteranninnenseite sowie am Rücken durchgeführt. Die Farbveränderung der Haut wurde während der Supplementierung durch ein Minolta-Chromameter (L-, a-, b-System) in Hautrötung (a-Werte), Gelbanteil (b-Werte) und Hauthelligkeit (L-Werte) differenziert. Die Konzentration an β-Carotin, Lycopin sowie Lutein im Serum wurde mittels Hochdruckflüssigkeitschromatographie (HPLC) bestimmt.

Die Ergebnisse sind in Tabelle 1 zusammengefasst und stellen die Mittelwerte für das Probandenpanel nach Abschluss der Untersuchung dar; die photoprotektive Wirkung wird gegenüber dem Blindwert (d.h. keine Zugabe von Carotinoiden, Gruppe 3) angegeben.

**Tabelle 1**

| **Photoprotektive Wirkung** | | |
|---|---|---|
| **Gruppe** | **Carotinoide (tägliche Dosis)** | **Photoprotektion [%-rel.]** |
| 1 | β-Carotin (24 mg) | 200 |
| 2 | β-Carotin (8 mg), Lycopin (8 mg) und Lutein (8 mg) | 270 |
| 3 | Kontrollgruppe ohne Carotinoide | 100 |

Man erkennt, dass gegenüber dem Blindwert (Gruppe 3) bei Dosierung der Betatenemischung (β-Carotin) die Photoprotektion der Haut verdoppelt wird (Gruppe 1). Setzt man Mischungen von Betatene (β-Carotin) und Lutein und Lycopin ein, kommt es zu einer deutlichen Steigerung der Photoprotektion (Gruppe 2). Die Ergebnisse der Gruppe 2 machen deutlich, dass es sich hierbei nicht um eine additive Wirkung handelt, da die gleiche Menge β-Carotin (Gruppe 3) diese protektive Wirkung nicht erreicht.

## Patentansprüche

1. Verwendung einer Zusammensetzung bestehend aus
(a) β-Carotin,
(b) Lutein und
(c) Lycopin
in einem Gewichtsverhältnis von (a) : (b) : (c) von 1: (0,5 -1,5) : (0,5 - 1,5) und einem für die orale Aufnahme geeigneten Träger zur Herstellung einer oralen therapeutischen Zubereitung zur Erhöhung des Lichtschutzfaktors der Haut.

2. Kosmetische Verwendung einer Zusammensetzung bestehend aus
(a) β-Carotin,
(b) Lutein und
(c) Lycopin
in einem Gewichtsverhältnis von (a) : (b) : (c) von 1: (0,5 -1,5) : (0,5 - 1,5) und einem für die orale Aufnahme geeigneten Träger zur Verzögerung des Alterungsprozesses der Haut durch orale Aufnahme dieser Zusammensetzung.

3. Verwendung nach den Ansprüchen 1 oder 2, **dadurch gekennzeichnet, dass** die Zubereitungen in einer Tagesdosis von
(a) 1 bis 40 mg β-Carotin,
(b) 1 bis 40 mg (b) Lutein und
(c) 1 bis 40 mg (c) Lycopin
mit der Maßgabe eingesetzt werden, dass das Verhältnis von (a) : (b) : (c) bei 1 : (0,5 - 1,5) : (0,5 - 1,5) liegt.

## Claims

1. The use of a composition consisting of
(a) β-carotene,
(b) lutein and
(c) lycopene
in a ratio by weight of (a) to (b) to (c) of 1 : (0.5 - 1.5) : (0.5 - 1.5) and a carrier suitable for oral administration for the production of an oral therapeutic preparation for increasing the sun protection factor of the skin.

2. The cosmetic use of a composition consisting of
(a) β-carotene,
(b) lutein and
(c) lycopene
in a ratio by weight of (a) to (b) to (c) of 1 : (0.5 - 1.5) : (0.5 - 1.5) and a carrier suitable for oral administration for delaying the ageing process of the skin by oral administration of the composition.

3. The use claimed in claim 1 or 2, **characterized in that** the preparations are used in a daily dose of
(a) 1 to 40 mg β-carotene,
(b) 1 to 40 mg (b) lutein and
(c) 1 to 40 mg (c) lycopene,
with the proviso that the ratio of (a) to (b) to (c) is 1 : (0.5 - 1.5) : (0.5 - 1.5).

## Revendications

1. Utilisation d'une composition constituée de
(a) β-carotène
(b) lutéine et
(c) lycopine
selon une proportion pondérale de (a) : (b) : (c) égale à 1 : (0,5 - 1,5) : (0,5 - 1,5) et d'un vecteur adapté pour une administration orale pour la fabrication d'une préparation thérapeutique orale destinée à augmenter l'indice de protection de la peau.

2. Utilisation cosmétique d'une préparation constituée de
(a) β-carotène
(b) lutéine et
(c) lycopine
selon une proportion pondérale de (a) : (b) : (c) égale à 1 : (0,5 - 1,5) : (0,5 - 1,5) et d'un vecteur adapté pour une administration orale pour le ralentissement du processus de vieillissement de la peau par l'intermédiaire de l'administration orale de cette préparation.

3. Utilisation selon l'une quelconque des revendications 1 ou 2, **caractérisée en ce que**
la préparation est utilisée selon une dose quotidienne de
(a) 1 à 40 mg de β-earotène
(b) 1 à 40 mg (b) de lutéine et
(c) 1 à 40 mg (c) de lycopine
de telle sorte que la proportion pondérale de (a) : (b) : (c) soit égale à 1 : (0,5 - 1,5) : (0,5 - 1,5).
